# FASCICULE DE BREVET EUROPEEN

(11) **EP 0 839 016 B1**
(45) Date de publication et mention de la délivrance du brevet: **04.12.2002**
(21) Numéro de dépôt: 97924098.3
(22) Date de dépôt: 14.05.1997
(51) Int. Cl.: A61F 2/34

(54) **IMPLANT COTYLOIDIEN FIXE SANS CIMENT**
ZEMENTFREI VERANKERTES HÜFTGELENKPFANNENIMPLANTAT
COTYLOIDAL IMPLANT FIXED WITHOUT CEMENT

(30) Priorité: 14.05.1996 FR 9606243; 14.05.1996 FR 9606244
(43) Date de publication de la demande: 06.05.1998
(73) Titulaire: Balay, Bruno, 01600 Saint Bernard (FR); Cartillier, Jean-Claude, 69008 Lyon (FR); Charlet, Claude, 69370 Saint Didier au Mont d'Or (FR); Machenaud, Alain, 74000 Cran-Gevrier (FR); Semay, Jean-Marc, 42270 Saint Priest en Jarest (FR); Setiey, Louis, 69400 Gleize (FR); Vidalain, Jean-Pierre, 74000 Annecy le Vieux (FR)
(72) Inventeur: Balay, Bruno, 01600 Saint Bernard (FR); Cartillier, Jean-Claude, 69008 Lyon (FR); Charlet, Claude, 69370 Saint Didier au Mont d'Or (FR); Machenaud, Alain, 74000 Cran-Gevrier (FR); Semay, Jean-Marc, 42270 Saint Priest en Jarest (FR); Setiey, Louis, 69400 Gleize (FR); Vidalain, Jean-Pierre, 74000 Annecy le Vieux (FR)
(74) Mandataire: Bernasconi, Jean
(86) Numéro de dépôt international: FR9700860
(87) Numéro de publication internationale: WO97042913

(56) Documents cités:
- EP-A- 0 149 975
- EP-A- 0 245 527
- EP-A- 0 341 198
- EP-A- 0 407 332
- EP-A- 0 444 381
- EP-A- 0 485 678
- EP-A- 0 677 280
- WO-A-95/22944
- DE-A- 4 337 936
- DE-A- 4 408 527
- FR-A- 2 630 907
- FR-A- 2 638 963
- FR-A- 2 660 546
- FR-A- 2 678 510
- GB-A- 2 268 408
- US-A- 4 892 549

## Description

La présente invention concerne un implant cotyloïdien fixable sans ciment.

Un implant cotyloïdien comprend classiquement une cupule de forme hémisphérique, délimitant une cavité intérieure, et un insert pouvant être engagé dans cette cavité. La cupule est destinée à être engagée dans la cavité cotyloïdienne et à être fixée au bassin, et l'insert délimite une cavité sphérique destinée à recevoir, avec possibilité de pivotement, la tête sphérique correspondante d'une tige fémorale.

La cupule est généralement en matériau métallique, notamment en titane, et l'insert est en matériau favorisant le glissement de la tête fémorale, par exemple en céramique d'alumine ou polyéthylène à haute densité.

Certains implants sont fixés au bassin au moyen de ciment synthétique polymérisable ; d'autres sont fixés sans ciment grâce à des moyens d'ancrage mécaniques tels que des vis.

Parmi ceux-ci, il existe des implants dits "press fit", destinés à être insérés à force dans la cavité cotyloïdienne. La cupule d'un tel implant présente un diamètre externe légèrement supérieur à celui de la cavité cotyloïdienne, notamment dans sa zone périphérique, et comprend des plots ou des aspérités venant s'insérer dans l'os lors de l'impaction de la cupule dans la cavité.

Ces plots ou aspérités ne suffisent généralement pas à assurer, à eux seuls, une fixation de la cupule parfaitement fiable dans le temps. C'est pourquoi ces implants comprennent en plus des vis insérées dans l'os au travers de la cupule pour assurer un ancrage complémentaire.

Certaines cupules comprennent en outre des fentes, et éventuellement des ouvertures, permettant de leur conférer une certaine souplesse radiale en vue de leur ancrage par expansion dans la cavité cotyloïdienne.

Les implants existants fixés sans ciment assurent un ancrage solide et fiable de la cupule dans le cotyle, mais ont toutefois pour inconvénient majeur de permettre la diffusion vers l'intérieur de la cavité cotyloïdienne et dans l'organisme de débris d'usure du matériau constituant l'insert.

Ces débris résultent du frottement de la tête fémorale dans l'insert et des micro-mouvements de l'insert dans la cupule. Ils résultent également du frottement de l'insert contre les arêtes plus ou moins vives qui délimitent les évidements, trous, fentes ou ouvertures précités de la cupule, et ce d'autant plus que l'insert a tendance à fluer dans ces évidements, trous, fentes ou ouvertures sous l'effet des contraintes répétées que subit la prothèse.

En outre, les trous, fentes ou ouvertures précités permettent la circulation vers l'os de liquide synovial chargé de particules, cette circulation résultant d'un "effet de pompe" généré par la succession de mises en pression et de relâchements de l'implant dans la cavité lorsque le patient marche.

Ces débris de polyéthylène provoquent une ostéolyse néfaste à la bonne tenue des vis dans le temps, et sont mal tolérés par l'organisme.

Un autre inconvénient des cupules "press fit" est le risque d'une liaison insuffisante entre la surface externe de la cupule et l'os iliaque, notamment au niveau des reliefs d'accrochage dans la zone équatoriale de la cupule, de sorte qu'il est fréquent de devoir utiliser des vis de fixation dans le tissu osseux, même pour les cupules revêtues d'un matériau d'aide à l'ostéo-intégration, tel que l'hydroxyapatite de calcium.

La présente invention vise à remédier à ces inconvénients, en fournissant un implant cotyloïdien pouvant être ancré au bassin sans ciment et sans recourir à la mise en place de vis.

L'implant qu'elle concerne comprend une cupule et un insert tels que précités, la cupule étant mise en place par impaction et comprenant, à cet effet, des aspérités d'ancrage aménagées sur sa face extérieure. Un implant montrant les caractéristiques du préambule de la revendication 1 est décrit dans GB-A-2 268 408 .

Selon l'invention, la cupule comprend, aménagées dans sa face extérieure, coaxialement à elle, deux rainures en forme de filets hélicoïdaux de pas inverses, qui s'entrecroisent l'une l'autre sur une large partie de cette face externe, qui sont inclinées en direction de l'ouverture de la cupule et qui présentent une profondeur augmentant progressivement en direction de l'ouverture de la cupule, ces rainures, par leur entrecroisement, délimitant un ensemble d'aspérités en forme de losange, contiguës et imbriquées.

Avantageusement ces aspérités sont régulièrement réparties sur la surface extérieure de la cupule et leurs dimensions décroissent progressivement vers la zone polaire de la cupule.

L'inclinaison de ces rainures et l'augmentation de leur profondeur au long de la face de la cupule permettent une insertion progressive des aspérités dans l'os, sur l'ensemble de la périphérie de la cupule, lors de l'impaction. Simultanément, le nombre, la disposition et le caractère acéré de ces aspérités assurent un parfait ancrage de la cupule dans l'os, rendant inutile la mise en place de vis complémentaires d'ancrage.

C'est en effet sur la partie du cotyle naturel la plus résistante mécaniquement que ces aspérités viennent s'accrocher et sont les plus efficaces. C'est pourquoi la profondeur des rainures est la plus importante sur la partie de la cupule contiguë à son plan équatorial, en regard de laquelle la résistance mécanique de la paroi osseuse est la plus élevée.

La cupule peut donc, dès lors, ne comprendre aucun trou pour le passage de telles vis, et aucune fente ou ouverture. L'absence de ces trous, fentes ou ouvertures permet d'éliminer le risque de fluage de l'insert et d'éliminer toute arête plus ou moins vive contre laquelle l'insert pourrait s'user.

L'usure de l'insert, et donc l'émission de particules, reste donc extrêmement limitée dans l'implant selon l'invention.

De plus, l'absence de ces trous, fentes ou ouvertures permet de piéger à l'intérieur de la cupule les éventuelles particules générées par l'usure de l'insert, et supprime, par conséquent, le risque de migration de ces particules vers l'os, au travers de la cupule.

Dans une forme de réalisation particulièrement préférée, la cupule présente une surface extérieure traitée ou revêtue de façon à favoriser la liaison par ostéo-intégration sur cette surface. Pour ce faire, elle reçoit un revêtement à base d'hydroxyapatite de calcium. Grâce à un revêtement de ce biomatériau sur la surface externe de la cupule, il devient possible, après avoir réalisé une fixation primaire par l'effet mécanique des moyens dits « Press-Fit » sur le bord osseux du cotyle, de réaliser une fixation secondaire de nature physico-chimique, qui vient renforcer la fixation première dans les premiers mois consécutifs à l'opération. Cette fixation est obtenue par adsorption des différents constituants du tissu osseux sur le dépôt d'hydroxyapatite de calcium couvrant la face externe de la cupule.

De préférence, les rainures sont inclinées en direction de l'ouverture de la cupule selon un angle de l'ordre de 45°, pour un parfait ancrage des aspérités dans l'os, sans possibilité de recul de ladite cupule.

Avantageusement, les flancs de deux aspérités délimitées par une même rainure sont asymétriques, le flanc situé du côté de l'ouverture de la cupule étant plus incliné vers le plan équatorial, par rapport à l'axe de la cupule, que le flanc situé du côté du fond de la cupule. Cette asymétrie favorise l'ancrage.

En effet, grâce à cette caractéristique avantageuse de l'invention, le flanc de rainure situé du côté de l'ouverture présente une surface supérieure à celle de l'autre flanc, et cette surface est relativement peu inclinée par rapport au plan tangent à la sphère, de sorte que les contraintes maximales exercées à ce niveau sur le tissu osseux sont surtout des contraintes de compression, ce qui favorise la croissance osseuse et l'ostéo-intégration de la prothèse dans cette zone délicate.

Dans le même but, les filets présentent avantageusement, en section transversale, une forme de "bec d'aigle", c'est-à-dire comprennent une arête d'extrémité acérée, légèrement recourbée en direction de l'ouverture de la cupule.

On préfère donc que la face extérieure de la cupule soit recouverte d'un revêtement favorisant l'ostéo-intégration, en particulier d'un revêtement d'hydroxyapatite de calcium. L'épaisseur de ce revêtement peut être, par exemple, de l'ordre de 70 à 150 µ.

En outre, la cupule peut comprendre un trou aménagé au niveau de son pôle pour la prise d'appui de l'outil d'impaction. Dans ce cas, ce trou est taraudé et reçoit un obturateur fileté venant, en fin de serrage, affleurer avec la face interne de la cupule.

Ce bouchon permet d'éliminer le risque de fluage de l'insert dans le trou et de conserver une continuité à la paroi de la cupule.

Dans une forme de réalisation préférée de l'invention, destinée à réduire au maximum le risque de formation et de propagation de débris d'insert, la prothèse peut être réalisée de façon que :
- la cupule présente une paroi continue, c'est-à-dire est dépourvue d'évidements, trous, fentes ou ouvertures, et présente une surface interne polie et parfaitement lisse,
- le trou aménagé pour la prise d'appui de l'outil d'impaction est taraudé et peut recevoir un obturateur fileté venant, en fin de serrage, affleurer avec la face interne de la cupule, et
- la partie de l'insert destinée à être engagée dans la cupule présente une forme complémentaire de celle de la cavité de la cupule et vient intimement au contact de la face interne de la cupule.

Ainsi, l'insert épouse la face interne lisse et continue de la cupule, ce qui permet de réduire au maximum les frottements et micro-mouvements de l'insert. L'absence d'évidements, de trous, de fentes ou d'ouvertures dans la paroi, après mise en place de l'obturateur fileté, permet d'éliminer toute arête plus ou moins vive contre laquelle l'insert pourrait s'user.

Il en résulte que l'usure de l'insert, et donc l'émission de particules, est extrêmement limitée dans l'implant selon l'invention. La large surface d'appui de l'insert contre la cupule limite également cette usure.

En outre, l'absence de trous, fentes ou ouvertures permet de piéger les éventuelles particules à l'intérieur de la cupule et supprime, par conséquent, le risque de migration de particules vers l'os, au travers de la cupule.

Avantageusement, la cavité de la cupule présente, du côté de son fond, une partie de forme parfaitement hémisphérique et, du côté de son ouverture, une partie de forme légèrement tronconique, ces deux parties étant séparées par un épaulement conique, et l'insert comprend une partie hémisphérique, une partie légèrement tronconique et une saillie de forme correspondant respectivement aux deux parties et à l'épaulement de la cavité de la cupule.

Les deux parties de l'insert viennent ainsi intimement au contact de la face interne de la cupule lorsque, après engagement à force de l'insert dans la cavité de la cupule, la saillie de l'insert est verrouillée derrière l'épaulement de ladite cupule.

Cette structure assure en outre une parfaite rétention de l'insert dans la cupule.

Selon un mode de réalisation de l'insert, celui-ci comprend un rebord anti-luxation, et l'implant comprend des moyens d'indexation pour le positionnement angulaire de ce rebord par rapport à la cupule, ces moyens d'indexation étant aménagés, d'une part, dans une collerette que comprend l'insert et, d'autre part, dans le bord de la cupule délimitant l'ouverture de la cavité.

Le rebord anti-luxation peut ainsi être orienté de manière adéquate en fonction de l'anatomie spécifique du patient, et les moyens d'indexation se trouvent en dehors de la cavité de la cupule. D'éventuelles émissions de particules générées au niveau de ces moyens d'indexation sont ainsi diffusées à l'extérieur de l'implant, et non à l'intérieur en direction de l'os.

Selon une forme de réalisation préférée de l'invention, ces moyens d'indexation sont constitués par une pluralité d'encoches radiales aménagées à distance régulière les unes des autres dans la périphérie de la collerette de l'insert et par des tétons aptes à être engagés dans ces encoches, aménagés sur la face annulaire équatoriale de la cupule.

Pour sa bonne compréhension, l'invention est à nouveau décrite ci-dessous en référence au dessin schématique annexé représentant, à titre d'exemple non limitatif, une forme de réalisation préférée de l'implant cotyloïdien qu'elle concerne.
La figure 1 est une vue en perspective éclatée de ses différents éléments constitutifs, avec arrachement partiel sur un élément;
la figure 2 est une vue de côté schématique de l'un de ces éléments;
la figure 3 est une vue partielle et à échelle agrandie de l'élément montré à la figure 2, en coupe selon l'axe de cet élément;
la figure 4 est une vue de ce même élément après implantation, en coupe selon l'axe de cet élément, et
la figure 5 est une vue de l'implant après mise en place, en coupe selon l'axe de cet implant.

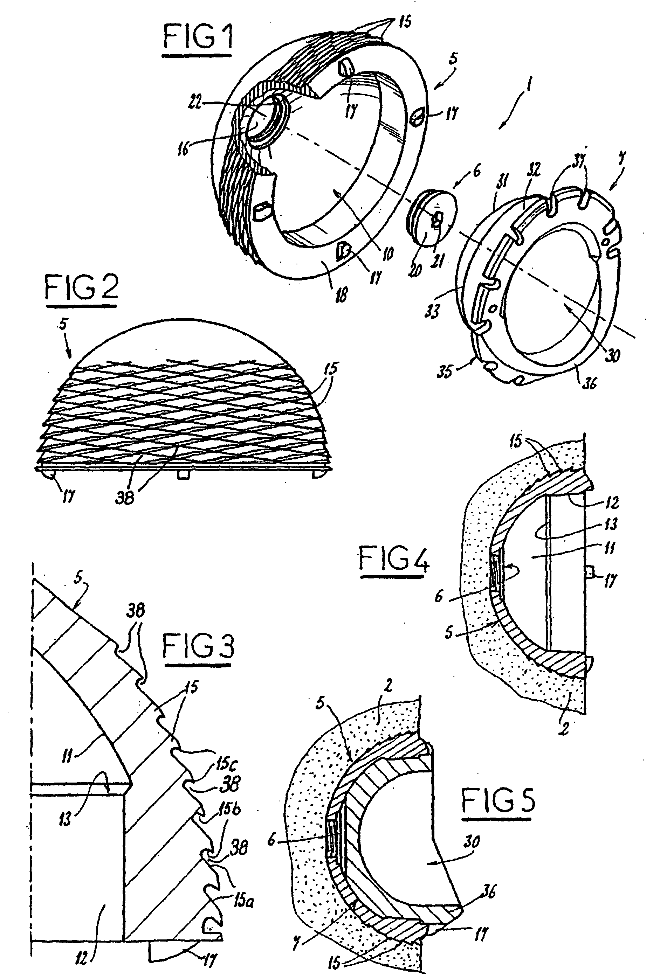

La figure 1 représente un implant cotyloïdien 1 de prothèse de hanche, destiné, ainsi que le montrent les figures 4 et 5, à être fixé sans ciment au bassin 2 du patient, en étant engagé dans la cavité cotyloïdienne.

L'implant 1 est constitué par une cupule 5 en matériau métallique, notamment en titane, par un bouchon fileté 6, en matériau métallique de même nature que la cupule 5, et par un insert 7 en matériau favorisant le glissement, notamment en polyéthylène à haute densité.

La cupule 5 présente une forme hémisphérique et délimite une cavité intérieure 10 dans laquelle peut être engagé l'insert 7.

La face intérieure de la cupule 5 présente, du côté du fond de cette cavité 10, une partie 11 de forme parfaitement hémisphérique et, du côté de l'ouverture de cette cavité 10, une partie 12 de forme légèrement tronconique, dont la plus faible section circulaire est située vers l'intérieur de la cavité 10. Ces deux parties 11, 12 sont séparées par un épaulement conique 13 dont la plus faible section est située du côté de l'ouverture de la cavité 10.

La cupule 2 présente par ailleurs un diamètre externe légèrement supérieur à celui de la cavité cotyloïdienne et est destinée à être ancrée par impaction dans celle-ci.

Elle comprend à cet effet, ainsi que le montrent plus particulièrement les figures 1 à 3, un nombre important d'aspérités 15 en forme de losange, contiguës et imbriquées, de préférence régulièrement réparties.

Ces aspérités 15 sont formées par l'entrecroisement, sur une large partie de la face externe de la cupule 5, de deux rainures 38 en forme de filets hélicoïdaux de pas inverses, aménagées coaxialement à la cupule 5. Il apparaît à la figure 3 que ces rainures 38 sont inclinées en direction de l'ouverture de la cupule 5, selon un angle de l'ordre de 45°, que leur profondeur augmente progressivement en direction de cette ouverture, et que les flancs 15a, 15b de deux aspérités 15 délimitées par une même rainure 38 sont asymétriques, le flanc 15a situé du côté de l'ouverture de la cupule 5 étant plus incliné par rapport à l'axe de la coiffe 5 et donc plus près du plan tangentiel à la sphère en cet endroit, que le flanc 15b situé du côté du fond de la cupule 5.

Les filets présentent, en section transversale, une forme en "bec d'aigle", c'est-à-dire comprennent une arête d'extrémité 15c acérée, légèrement recourbée en direction de l'ouverture de la cupule 5.

La surface externe de la cupule, y compris les flancs 15a des filets, est revêtue d'une couche d'hydroxyapatite de calcium, par toute technique bien connue de l'homme de l'art dans le domaine des prothèses métalliques destinées à l'ostéointégration, par exemple par le procédé APS (atmospheric plasma spraying) utilisant une torche à plasma.

La cupule 5 comprend également un trou taraudé 16 aménagé au niveau de son pôle, pour la prise d'appui de l'outil d'impaction, et quatre tétons 17 aménagés à égale distance les uns des autres sur sa face annulaire équatoriale 18 délimitant l'ouverture de la cavité 10.

Le bouchon fileté 6 est conformé pour pouvoir être vissé à l'intérieur du trou 16. Il présente une tête annulaire 20 dans laquelle est aménagée une cavité 21 à six pans, pour sa manoeuvre en rotation et son serrage au moyen d'une clef appropriée. Cette tête 20 vient s'engager, en fin de vissage, dans un évidement annulaire 22 coaxial au trou 16, et vient par conséquent, ainsi que le montre la figure 4, affleurer avec la face interne de la cupule 5.

L'insert 7 délimite une cavité sphérique 30 destinée à recevoir, avec possibilité de pivotement, la tête sphérique correspondante (non représentée) d'une tige fémorale, afin de permettre le jeu de l'articulation prothétique.

Il comprend une partie hémisphérique 31, une partie légèrement tronconique 32 et une saillie 33 de forme correspondant respectivement aux parties 11, 12 et à l'épaulement 13 de la cavité 10. Ces parties 31 et 32 viennent ainsi intimement au contact de la face interne de la cupule 5 lorsque, après engagement en force de l'insert 7 dans la cavité 10, la saillie 33 est verrouillée derrière l'épaulement 13.

En outre, l'insert 7 comprend une collerette 35 venant, comme le montre la figure 5, au contact de la face 18 lorsque l'insert 7 est verrouillée dans la cavité 10.

Cette collerette 35 comprend un rebord 36 anti-luxation de la tête fémorale, et présente une pluralité d'encoches radiales 37, aménagées à distance régulière les unes des autres dans sa périphérie. Ces encoches peuvent être engagées autour des tétons 17.

En pratique, la cupule 5 est montée sur la tige d'un instrument d'impaction (non représenté) puis est impactée dans la cavité cotyloïdienne du bassin 2, de manière à ce que ses aspérités 15 viennent d'insérer dans la paroi osseuse.

Lors de cette impaction, les aspérités 15 s'insèrent progressivement dans l'os, sur l'ensemble de la périphérie de la cupule 5. Le nombre, la forme, la disposition et le caractère acéré de ces aspérités 15 assurent un parfait ancrage de la cupule 5 dans l'os, sans possibilité de recul.

La bonne venue de la cupule 5 au contact du fond de la cavité peut être vérifiée grâce au trou 16, après retrait de l'outil d'impaction.

Le bouchon 6 est ensuite vissé dans le trou 16, puis l'insert 7 est partiellement engagé dans la cavité 10 et est positionné angulairement par rapport à la cupule 5 pour orienter au mieux le rebord 36 en fonction de l'anatomie spécifique du patient. Une fois ce positionnement effectué, l'insert 7 est engagé en force dans la cavité 10, jusqu'à verrouillage de sa saillie 33 derrière l'épaulement 13.

L'absence de trous, de fentes ou d'ouvertures dans la paroi de la cupule 2, après la mise en place du bouchon 16, élimine le risque de fluage de l'insert 7 et élimine toute arête plus ou moins vive contre laquelle cet insert pourrait fluer. De plus, l'insert 7 épouse intimement la face interne lisse et continue de la cupule 5.

Il en résulte que les frottements et micro-mouvements de l'insert 7 par rapport à la cupule 5 sont réduits au maximum et que l'émission de particules d'usure est extrêmement limitée.

Les éventuelles particules d'usure sont piégées à l'intérieur de la cupule 5, ce qui supprime le risque d'une migration de ces particules vers l'os, au travers de la cupule 5.

Par ailleurs, d'éventuelles particules générées au niveau des encoches 37 sont diffusées à l'extérieur de l'implant 1, et non en direction de l'os.

La liaison par ostéo-intégration, qui débute quelques semaines après la pose, devient effective au bout de quelques mois à un an.

## Revendications

1. Implant cotyloïdien fixable sans ciment, comprenant une cupule (5) de forme hémisphérique, délimitant une cavité intérieure (10), et un insert (7) pouvant être engagé dans cette cavité (10), la cupule (5) étant mise en place par impaction et comprenant, à cet effet, des aspérités d'ancrage (15) aménagées sur sa face extérieure, qui présentent une forme de losange et qui sont contiguës et imbriquées, implant (1) **caractérisé en ce que** la cupule (5) comprend, aménagées dans sa face extérieure, coaxialement à elle, deux rainures en forme de filets hélicoïdaux (38) de pas inverses, qui s'entrecroisent l'une l'autre sur une large partie de cette face externe, qui sont inclinées en direction de l'ouverture de la cupule (5) et qui présentent une profondeur augmentant progressivement en direction de l'ouverture de la cupule (5), ces rainures, par leur entrecroisement, délimitant un nombre important d'aspérités (15).

2. Implant cotyloïdien selon la revendication 1, **caractérisé en ce que** les rainures (38) sont inclinées en direction de l'ouverture de la cupule (5) selon un angle de l'ordre de 45°.

3. Implant cotyloïdien selon la revendication 1 ou la revendication 2, **caractérisé en ce que** les flancs (15a, 15b) de deux aspérités (15) délimitées par une même rainure (38) sont asymétriques, le flanc (15a) situé du côté de l'ouverture de la cupule (5) étant plus incliné, par rapport à l'axe de la cupule (5), que le flanc (15b) situé du côté du fond de la cupule (5).

4. Implant cotyloïdien selon l'une des revendications 1 à 3, **caractérisé en ce que** les filets (38) présentent, en section transversale, une forme en "bec d'aigle", c'est-à-dire comprennent une arête d'extrémité acérée (15 c), légèrement recourbée en direction de l'ouverture de la cupule (5).

5. Implant selon l'une des revendications 1 à 4, **caractérisé en ce que** les aspérités (15) sont régulièrement réparties sur la surface extérieure de la cupule (5), et leurs dimensions décroissent progressivement vers la zone polaire de la cupule.

6. Implant cotyloïdien selon l'une des revendications 1 à 5, **caractérisé en ce que** la face extérieure de la cupule (5) est recouverte d'un revêtement favorisant l'ostéo-intégration, notamment d'un revêtement d'hydroxyapatite de calcium.

7. Implant cotyloïdien selon l'une des revendications 1 à 6, **caractérisé en ce que** la cupule (5) comprend un trou (16) aménagé au niveau de son pôle pour la prise d'appui d'un outil d'impaction, ce trou (16) étant taraudé et recevant un obturateur fileté (6) venant, en fin de serrage, affleurer avec la face interne de la cupule (5).

8. Implant cotyloïdien selon l'une des revendications 1 à 7, **caractérisé en ce que** :
- la cupule (5) présente une paroi continue, c'est-à-dire est dépourvue d'évidements, trous, fentes ou ouvertures, et présente une surface interne polie et parfaitement lisse,
- un trou (16) aménagé pour la prise d'appui de l'outil d'impaction est taraudé et peut recevoir un bouchon fileté (6) venant, en fin de serrage, affleurer avec la face interne de la cupule (5), et
- la partie (31, 32, 33) de l'insert (7) destinée à être engagée dans la cupule (5) présente une forme complémentaire de celle de la cavité (10) de la cupule (5) et vient intimement au contact de la face interne de la cupule (5).

9. Implant cotyloïdien selon l'une des revendications 1 à 8, **caractérisé en ce qu'**il comprend un insert (7) muni d'un rebord anti-luxation (36), et **en ce qu'**il comprend des moyens d'indexation (17, 37) pour le positionnement angulaire de ce rebord (36) par rapport à la cupule (5), ces moyens d'indexation (17, 37) étant aménagés, d'une part, dans une collerette (35) que comprend l'insert (7) et, d'autre part, dans la surface (18) de la cupule (5) délimitant l'ouverture de la cavité (10).

10. Implant cotyloïdien selon la revendication 9, **caractérisé en ce que** les moyens d'indexation sont constitués par une pluralité d'encoches radiales (37) aménagées à distance régulière les unes des autres dans la périphérie de la collerette (35) et par des tétons (17) aptes à être engagés dans ces encoches (37), aménagés sur la face annulaire équatoriale (18) de la cupule (5).

11. Implant cotyloïdien selon l'une des revendications 1 à 10, **caractérisé en ce que** la cavité (10) de la cupule (5) présente, du côté de son fond, une partie (11) de forme parfaitement hémisphérique et, du côté de son ouverture, une partie (12) de forme légèrement tronconique, ces deux parties (11, 12) étant séparées par un épaulement conique (13), et **en ce que** l'insert (7) comprend une partie hémisphérique (31), une partie légèrement tronconique (32) et une saillie (33) de forme correspondant respectivement aux deux parties (11, 12) et à l'épaulement (13) de la cavité (10) de la cupule (5).

## Patentansprüche

1. Gelenkpfannen-Implantat, das ohne Zement befestigbar ist und einen halbkugelförmigen Becher (5), der einen inneren Hohlraum (10) begrenzt, sowie einen Einsatz (7), der in diesem Hohlraum (10) in Eingriff gebracht werden kann, umfaßt, wobei der Becher (5) durch Einklemmen angeordnet wird und hierzu auf seiner äußeren Fläche ausgebildete Verankerungsunebenheiten (15) umfaßt, die Rautenform besitzen, aneinander angrenzen und verschachtelt sind, wobei das Implantat (1) **dadurch gekennzeichnet ist, daß** der Becher (5) zwei Rillen in Form Schraubenlinienförmiger Gewinde (38) mit entgegengesetzter Steigung umfaßt, die in seiner äußeren Fläche koaxial zu ihm ausgebildet sind, sich auf einem großen Teil dieser äußeren Fläche schneiden, in Richtung zur Öffnung des Bechers (5) geneigt sind und eine Tiefe aufweisen, die in Richtung zur Öffnung des Bechers (5) allmählich zunimmt, wobei diese Rillen durch ihre gegenseitige Verflechtung eine große Zahl von Unebenheiten (15) begrenzen.

2. Gelenkpfannen-Implantat nach Anspruch 1, **dadurch gekennzeichnet, daß** die Rillen (38) in Richtung zur Öffnung des Bechers (5) unter einem Winkel in der Größenordnung von 45 ° geneigt sind.

3. Gelenkpfannen-Implantat nach Anspruch 1 oder Anspruch 2, **dadurch gekennzeichnet, daß** die Seitenflächen (15a, 15b) zweier Unebenheiten (15), die durch dieselbe Rille (38) begrenzt sind, asymmetrisch sind, wobei die Seitenfläche (15a), die sich auf seiten der Öffnung des Bechers (5) befindet, in bezug auf die Achse des Bechers (5) stärker geneigt ist als die Seitenfläche (15b), die sich auf seiten des Bodens des Bechers (5) befindet.

4. Gelenkpfannen-Implantat nach einem der Ansprüche 1 bis 3, **dadurch gekennzeichnet, daß** die Gewinde (38) im Querschnitt die Form eines "Adlerschnabels" besitzen, d. h. sie besitzen eine scharfe Endkante (15c), die leicht in Richtung zur Öffnung des Bechers (5) gekrümmt ist.

5. Implantat nach einem der Ansprüche 1 bis 4, **dadurch gekennzeichnet, daß** die Unebenheiten (15) auf der äußeren Oberfläche des Bechers (5) regelmäßig verteilt sind und ihre Abmessungen zur polaren Zone des Bechers allmählich abnehmen.

6. Gelenkpfannen-Implantat nach einem der Ansprüche 1 bis 5, **dadurch gekennzeichnet, daß** die äußere Fläche des Bechers (5) mit einer die Osteointegration begünstigenden Beschichtung, insbesondere mit einer Calcium-Hydroxyapatit-Beschichtung, bedeckt ist.

7. Gelenkpfannen-Implantat nach einem der Ansprüche 1 bis 6, **dadurch gekennzeichnet, daß** der Becher (5) ein Loch (16) aufweist, das auf Höhe seines Pols ausgebildet ist, um darauf ein Einklemmwerkzeug abzustützen, wobei dieses Loch (16) mit Gewinde versehen ist und einen Gewindeverschluß (6) aufnimmt, der am Ende des Klemmvorgangs mit der inneren Fläche des Bechers (5) bündig abschließt.

8. Gelenkpfannen-Implantat nach einem der Ansprüche 1 bis 7, **dadurch gekennzeichnet, daß**:
- der Becher (5) eine ununterbrochene Wand, d. h. eine Wand, die keine Vertiefungen, Löcher, Schlitze oder Öffnungen besitzt, sowie eine innere Oberfläche, die poliert und vollkommen glatt ist, aufweist,
- ein Loch (16), das für die Abstützung des Einklemmwerkzeugs ausgebildet ist, mit Gewinde versehen ist und einen Gewindestopfen (6) aufnehmen kann, der am Ende des Klemmvorgangs mit der inneren Fläche des Bechers (5) bündig abschließt, und
- der Teil (31, 32, 33) des Einsatzes (7), der dazu bestimmt ist, in dem Becher (5) in Eingriff zu gelangen, eine zu jener des Hohlraums (10) des Bechers (5) komplementäre Form aufweist und in engen Kontakt mit der inneren Fläche des Bechers (5) gelangt.

9. Gelenkpfannen-Implantat nach einem der Ansprüche 1 bis 8, **dadurch gekennzeichnet, daß** es einen Einsatz (7) umfaßt, der mit einer Luxationsverhinderungseinfassung (36) versehen ist, und daß es Indexierungsmittel (17, 37) für die Winkelpositionierung dieser Einfassung (36) in bezug auf den Becher (5) umfaßt, wobei diese Indexierungsmittel (17, 37) einerseits in einem Kranz (35), den der Einsatz (7) umfaßt, und andererseits in der Oberfläche (18) des Bechers (5) ausgebildet sind und die Öffnung des Hohlraums (10) begrenzen.

10. Gelenkpfannen-Implantat nach Anspruch 9, **dadurch gekennzeichnet, daß** die Indexierungsmittel aus mehreren radialen Kerben (37), die in regelmäßigem gegenseitigen Abstand im Umfang des Kranzes (35) ausgebildet sind, und aus mehreren Zapfen (17), die in diesen Kerben (37) in Eingriff gelangen können und auf der ringförmigen Äquatorialfläche (18) des Bechers (5) ausgebildet sind, gebildet sind.

11. Gelenkpfannen-Implantat nach einem der Ansprüche 1 bis 10, **dadurch gekennzeichnet, daß** der Hohlraum (10) des Bechers (5) auf seiten seines Bodens einen Teil (11), der vollkommen halbkugelförmig ist, und auf seiten seiner Öffnung einen Teil (12) mit leicht kegelstumpfförmiger Gestalt aufweist, wobei diese beiden Teile (11, 12) durch eine konische Schulter (13) getrennt sind, und daß der Einsatz (7) einen halbkugelförmigen Teil (31), einen leicht kegelstumpfförmigen Teil (32) und einen Vorsprung (33) mit jeweiligen Formen, die den beiden Teilen (11, 12) bzw. der Schulter (13) des Hohlraums (10) des Bechers (5) entsprechen, aufweist.

## Claims

1. A cotyloidal implant which can be fixed without cement comprising a cup (5) of hemispherical shape delimiting an internal cavity (10) and an insert (7) which can be engaged into said cavity (10), the cup (5) being set in place by impaction and comprising for that purpose anchoring roughness portions (5) provided on its external face, which are of a lozenge shape and which are contiguous and overlapping, the implant (1) being **characterised in that** the cup (5) comprises, provided in its external face and in coaxial relationship therewith, two grooves in the form of helical threads (38) of opposite pitches which intersect each other over a large part of said external face, which are inclined in the direction of the opening of the cup (5) and which are of a depth that increases progressively in the direction of the opening of the cup (5), said grooves by virtue of their intersection delimiting a substantial number of roughness portions (15).

2. A cotyloidal implant according to claim 1 **characterised in that** the grooves (38) are inclined in the direction of the opening of the cup (5) at an angle of the order of 45°.

3. A cotyloidal implant according to claim 1 or claim 2 **characterised in that** the flanks (15a, 15b) of two roughness portions (15) which are delimited by the same groove (38) are asymmetrical, the flank (15a) which is disposed at the side of the opening of the cup (5) being more inclined with respect to the axis of the cup (5) than the flank (15b) which is disposed at the side of the bottom of the cup (5).

4. A cotyloidal implant according to one of claims 1 to 3 **characterised in that** the threads (38) are in the shape of an 'eagle's beak' in cross-section, that is to say comprising a sharp-pointed end edge (15c) which is slightly curved in the direction of the opening of the cup (5).

5. An implant according to one of claims 1 to 4 **characterised in that** the roughness portions (15) are regularly distributed over the external surface of the cup (5) and their dimensions progressively decrease towards the polar zone of the cup.

6. A cotyloidal implant according to one of claims 1 to 5 **characterised in that** the external face of the cup (5) is covered with a coating for promoting osteo-integration, in particular a coating of calcium hydroxyapatite.

7. A cotyloidal implant according to one of claims 1 to 6 **characterised in that** the cup (5) comprises a hole (16) disposed at the location of its pole for the engagement of an impaction tool, said hole (16) being threaded and receiving a screwthreaded closure member (6) which at the end of being tightened is flush with the internal face of the cup (5).

8. A cotyloidal implant according to one of claims 1 to 7 **characterised in that**:
- the cup (5) has a continuous wall, that is to say it is devoid of recesses, holes, slots or openings, and has a polished and perfectly smooth internal surface,
- a hole (16) provided for engagement of the impaction tool is threaded and can receive a screwthreaded plug (6) which at the end of being tightened is flush with the internal face of the cup (5), and
- the portion (31, 32, 33) of the insert (7) which is intended to be engaged into the cup (5) is of a shape complementary to that of the cavity (10) of the cup (5) and comes intimately into contact with the internal face of the cup (5).

9. A cotyloidal implant according to one of claims 1 to 8 **characterised in that** it comprises an insert (7) provided with an anti-luxation rim (36) and that it comprises indexing means (17, 37) for angular positioning of said rim (36) with respect to the cup (5), said indexing means (17, 37) being provided on the one hand in a collar (35) that the insert (7) comprises and on the other hand in the surface (18) of the cup (5), which delimits the opening of the cavity (10).

10. A cotyloidal implant according to claim 9 **characterised in that** the indexing means are formed by a plurality of radial notches (37) provided at regular spacings from each other in the periphery of the collar (35) and by lugs (17) capable of being engaged into said notches (37) and provided on the equatorial annular face (18) of the cup (5).

11. A cotyloidal implant according to one of claims 1 to 10 **characterised in that** towards its bottom the cavity (10) of the cup (5) has a portion (11) of perfectly hemispherical shape and towards its opening it has a portion (12) of slightly frustoconical shape, said two portions (11, 12) being separated by a conical shoulder (13), and that the insert (7) comprises a hemispherical portion (31), a slightly frustoconical portion (32) and a projecting portion (33) of a shape corresponding respectively to the two portions (11, 12) and to the shoulder (13) of the cavity (10) of the cup (5).
